# EUROPÄISCHE PATENTANMELDUNG

(11) **EP 1 369 475 A1**
(43) Veröffentlichungstag der Anmeldung: **10.12.2003**
(21) Anmeldenummer: 03011810.3
(22) Anmeldetag: 26.05.2003
(51) Int. Cl.: C12N 9/02, C12P 7/22

(54) **Mikrobiologisches Verfahren zur enantioselektiven (S)-Hydroxylierung**

(30) Priorität: 06.06.2002 DE 10225371
(71) Anmelder: Bayer Aktiengesellschaft, 51368 Leverkusen (DE)
(72) Erfinder: Berendes, Frank, Dr., 50823 Köln (DE); Krzossok, Nicola, 68305 Mannheim (DE)

(57) **Zusammenfassung**

Die Erfindung betrifft ein mikrobiologisches Verfahren zur Herstellung chiraler Arylalkanole durch enantioselektive Hydroxylierung von Arylalkanen in Gegenwart von Wirtszellen eines Mikroorganismus, die die Gene von Oxygenasen exprimieren.

## Beschreibung

Die Erfindung betrifft ein mikrobiologisches Verfahren zur Herstellung chiraler Arylalkanole durch enantioselektive Hydroxylierung von Arylalkanen in Gegenwart von Wirtszellen eines Mikroorganismus, die die Gene von Oxygenasen exprimieren.

Es ist bekannt, dass die biochemische Oxidation von aromatischen und aliphatischen Verbindungen durch Oxygenasen erfolgen kann, die von Mikroorganismen gebildet werden. Aliphatische Verbindungen können dabei zu primären und sekundären Alkoholen oxidiert werden (W. A. Duetz et al., Current Opinion in Biotechnology, 12 (2001) 419-425; H. L. Holland, H. K. Weber, Current Opinion in Biotechnology, 11 (2000) 547-553; H. L. Holland, Current Opinion in Biotechnology, 3 (1999) 22-27).

Aus EP-A 277 674 ist ein mikrobiologisches Verfahren zur terminalen Hydroxylierung von apolaren aliphatischen Verbindungen mit 6 bis 12 C-Atomen bekannt.

In EP-A 502 524 und EP-A 477 828 werden mikrobiologische Verfahren zur terminalen Hydroxylierung von Methyl- und Ethylgruppen an 5- oder 6-Ring-Heterocyclen beschrieben. Zur Katalyse werden rekombinante *Escherichia coli-* oder *Pseudomonas-Stämme* verwendet, in denen die *xyl*-Gene aus *Pseudomonas putida* bzw. die *alk*-Gene aus *Pseudomonas oleovorans* exprimiert werden.

Verschiedene Arbeiten beschäftigen sich mit der Seitenkettenhydroxylierung von Alkylaromaten unter Verwendung von Bakterienstämmen, wobei jedoch hohe Enantioselektivitäten nur in Ausnahmefällen erreicht wurden und dann mit schlechten Regioselektivitäten verbunden waren (W. Adam, Z. Lukacs, C. Kahle, C. R. Saha-Möller, P. Schreier, Journal of Molecular Catalysis B: Enzymatic 11 (2001) 377-385).

In Uzura et al., Journal of Bioscience and Bioengeneering, Vol. 91, No. 2 (2001) 217-221 wird die enantioselektive Synthese von (R)-1-Phenylethanol und (R)-1-Phenylpropanol aus den entsprechenden Alkylbenzolen unter Verwendung von Pilzen und Hefen mit Enantiomerenüberschüssen von > 99 % (R) beschrieben. Es gelang aber ausschließlich die Synthese der (R)-Enantiomeren. Die Mikroorganismen wurden in einem breit angelegten Screening identifiziert.

K. Lee und D.T. Gibson beschreiben in Appl. Environ. Microbiol., Vol. 62, No. 9 (1996) 3101-3106, dass durch die Naphthalin-Dioxygenase (NDO) aus dem Stamm *Pseudomonas sp.* NCIB 9816-4 Ethylbenzol zu einem Gemisch aus 1-Phenylethanol, Acetophenon, 2-Hydroxyacetophenon, Styrol und 1-Phenyl-1,2-ethandiol umgesetzt werden kann. Phenylethanol lag dabei mit einem Enantiomerenüberschuß (ee) von 77 % als (S)-1-Phenylethanol vor. Die Reaktion wurde mit isolierten Enzymen durchgeführt, wobei sich sowohl die hohe Zahl an Nebenprodukten als auch die geringe Enantioselektivität nachteilig auf die Ausbeute an (S)-1-Phenylethanol auswirkte.

Eine enantioselektive enzymatische Synthese von (S)-1-Arylalkanolen mit hohen Enantiomerenüberschüssen wurde bislang nicht beschrieben.

Bei chiralen Alkoholen handelt es sich um wichtige Zwischenprodukte bei der Herstellung von pharmazeutischen Wirkstoffen, so dass die gezielte Darstellung der einzelnen Enantiomeren von großer Bedeutung ist.

Daher bestand die Aufgabe der vorliegenden Erfindung darin, ein einfaches Verfahren zur enantioselektiven Synthese von (S)-1-Arylalkanolen mit hohen Enantiomerenüberschüssen bereitzustellen.

Überraschend wurde nun das im Folgenden beschriebene erfindungsgemäße Verfahren gefunden, welches erstmals die Möglichkeit bietet, (S)-1-Homo- oder (S)-1-Heteroarylalkan-1-ole mit hohen Enantiomerenüberschüssen herzustellen.

Gegenstand der vorliegenden Erfindung ist daher ein Verfahren zur Herstellung von (S)-1-Homo- oder (S)-1-Heteroarylalkan-1-olen durch enantioselektive Hydroxylierung von 1-Homo- oder 1-Heteroarylalkanen, welches dadurch gekeiinzeichnet ist, dass die Hydroxylierung in Gegenwart eines Mikroorganismus durchführt wird, enthaltend Nukleinsäuren, die für eine Oxygenase kodieren, welche die enantioselektive (S)-Hydroxylierung katalysiert.

Das erfindungsgemäße Verfahren kann sowohl in Gegenwart eines Wildtyps als auch in Gegenwart eines transformierten Mikroorganismus durchgeführt werden.

Bevorzugt ist dies ein Verfahren, welches dadurch gekennzeichnet ist, dass die Hydroxylierung in Gegenwart eines transformierten Mikroorganismus durchführt wird, enthaltend Nukleinsäuren, die für eine Oxygenase kodieren.

Weiterhin bevorzugt ist dies ein Verfahren, welches dadurch gekennzeichnet ist, dass die Hydroxylierung in Gegenwart von Bakterien, Hefen oder Pilzen durchgeführt wird, enthaltend Nukleinsäuren, die für eine Oxygenase kodieren.

Besonders bevorzugt ist dies ein Verfahren, welches dadurch gekennzeichnet ist, dass die Hydroxylierung in Gegenwart von Gram-negativen Bakterien, insbesondere solchen der Gattung *Pseudomonas, Rhodopseudomonas, Burkholderia, Ralstonia, Comamonas, Acinetobacter, Rhizobium* oder *Escherichia coli,* durchgeführt wird.

Ganz besonders bevorzugt ist dies ein Verfahren, welches dadurch gekennzeichnet ist, dass die Hydroxylierung in Gegenwart eines *Escherichia coli*-K 12-Stammes oder eines *Pseudomonas putida*-Stammes durchgeführt wird.

Das erfindungsgemäße Verfahren wird in Gegenwart der vorangehend aufgeführten Mikroorganismen durchgeführt, welche Nukleinsäuren enthalten, die für eine Oxygenase kodieren. Unter Oxygenasen sind gemäß der vorliegenden Erfindung alle Proteine zu verstehen, die geeignet sind, Oxidationsreaktionen zu katalysieren, bei denen Sauerstoffatome direkt in das Substrat- oder Eduktmolekül eingebaut werden.

Bevorzugt wird das Verfahren gemäß der vorliegenden Erfindung in Gegenwart eines Mikroorganismus durchgeführt enthaltend Nukleinsäuren, die für eine Naphthalin-Dioxygenase kodieren.

Besonders bevorzugt wird das Verfahren durchgeführt in Gegenwart eines Mikroorganismus enthaltend Nukleinsäuren, die für eine Naphthalin-Dioxygenase aus *Pseudomonas putida G7* (DSM 4476) oder *Pseudomonas sp.* NCIB 9816 (DSM8368) oder ähnliche Enzyme mit mindestens 70 % Identität, bevorzugt 80% Identität, besonders bevorzugt 90% Identität über die Gesamtlänge der Aminosäuresequenz kodieren. Die entsprechende Sequenz der Naphthalin-Dioxygenase aus *Pseudomonas putida G7* (DSM 4476) oder *Pseudomonas sp.* NCIB 9816 (DSM 8368) ist beschrieben unter Seq ID: NCBI Acc. No. M83949: von Basenpaar 853 bis Basenpaar 4316.

Der Grad der Identität der Aminosäuresequenz wird vorzugsweise bestimmt mit Hilfe des Programms GAP aus dem Progrämmpaket GCG, Version 9.1 unter Standardeinstellungen (Devereux et al. (1984), Nucleic Acids Research 12, 387)

Ein im erfindungsgemäßen Verfahren bevorzugt eingesetzter transformierter Mikroorganismus kann für die Genexpression Expressionsvektoren aufweisen. Die Expressionsvektoren besitzen Sequenzen, die funktionell mit den zu exprimierenden Oxygenasegenen verknüpft sind, wie beispielsweise mindestens einen Promotor zur konstitutiven oder induzierbaren Expression oder auch Enhancer.

Bevorzugt sind Expressionsvektoren, die eine Expression der Oxygenasegene in Gram-negativen Bakterien gewährleisten, wie z.B. pUC18, pUC19, pVLT31, pVLT33, pVLT35, pProEXHTabc (Invitrogen, Karlsruhe), TOPO-Expressionsvektoren z.B. pCRT7/CT-TOPO (Invitrogen, Karlsruhe), Vektoren der pET-Serie beispielsweise pET3a, pET23a, pET28a, pET32a (Novagen, Bad Soden), Vektoren der pASK-Serie z.B. pASK-IBA7 (IBA, Göttingen), Vektoren der pQE-Serie z.B. pQE30, pQE31, pQE70 und pREP4 (Qiagen, Hilden), Vektoren der pBlueskript-Serie (Stratagen, Heidelberg) u.a.. Die Vektoren können optional N-terminale bzw. C-terminale Tags wie z.B. Strep-Tag oder His-Tag o.a. aufweisen.

Besonders bevorzugt sind die Expressionsvektoren pVLT33 und pCRT7/CT-TOPO.

Das erfindungsgemäße Verfahren ermöglicht die Herstellung von (S)-1-Homo- oder (S)-1-Heteroarylalkan-1-olen, im Folgenden zusammenfassend auch bezeichnet als (S)-1-Arylalkan-1-ole, durch enantioselektive Hydroxylierung von 1-Homo- oder 1-Heteroarylalkanen.

Bevorzugt werden im erfindungsgemäßen Verfahren als Edukte (C₁-C₆)-Alkyl-(C₅-C₁₄)-aromaten, insbesondere bevorzugt (C₁-C₆)-Alkyl-(C₅-C₆)-aromaten, eingesetzt werden, die einfach oder mehrfach, gleich oder verschieden ringsubstituiert sind und worin 0 bis 3 Kohlenstoffatome, d.h. 0, 1, oder 3 Kohlenstoffatome, durch Heteroatome aus der Gruppe N, O und/oder S im aromatischen Ringsystem ersetzt sind.

Besonders bevorzugt werden durch das erfindungsgemäße Verfahren (S)-1- Homoarylalkan-1-ole der allgemeinen Formel (I), 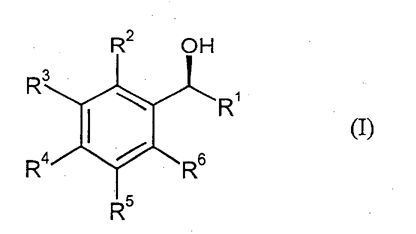 wobei
- R¹: für C₁-C₃-Alkyl, insbesondere bevorzugt für Methyl, steht,
- R², R³, R⁴, R⁵ und R⁶: unabhängig voneinander für H, Halogen, C₁-C₄-Halogenalkyl, C₁-C₄-Alkyl, Hydroxy, C₁-C₄-Alkoxy, Thiol, C₁-C₄-Thioalkoxy, Amino, primäres oder sekundäres C₁-C₄-Aminoalkyl stehen und mindestens ein Rest R² bis R⁶ von H verschieden ist, insbesondere bevorzugt unabhängig voneinander für H, Halogen, C₁-C₄-Halogenakyl stehen und mindestens ein Rest R² bis R⁶ von H verschieden ist,
durch enantioselektive Hydroxylierung von Verbindungen der allgemeinen Formel (II), 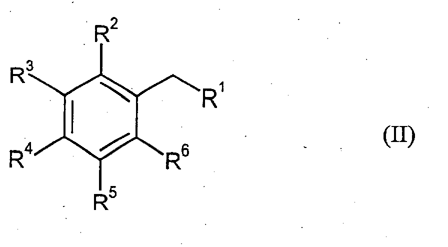 wobei
- R¹: die für Formel I genannte Bedeutung hat und
- R², R³, R⁴, R⁵ und R⁶: unabhängig voneinander die für Formel I genannte Bedeutung haben,
hergestellt.

C₁-C₃-Alkyl kann im Sinne der Erfindung Methyl, Ethyl, n-Propyl und iso-Propyl sein, C₁-C₄-Alkyl kann Methyl, Ethyl, n-Propyl, iso-Propyl, n-Butyl, tert-Butyl, isoButyl und sec-Butyl sein, C₁-C₄-Halogenalkyl kann einfach oder mehrfach Fluor-, Chlor-, Brom-und/oder Iod-substituiertes vorangehend aufgeführtes C₁-C₄-Alkyl sein, primäres oder sekundäres C₁-C₄-Aminoalkyl kann vorangehend aufgeführtes C₁-C₄-Alkyl mit entsprechenden Aminosubstituenten sein, C₁-C₄-Alkoxy kann Methoxy, Ethoxy, n-Propoxy, iso-Propoxy, n-Butoxy, tert-Butoxy, iso-Butoxy und sec-Butoxy sein und C₁-C₄-Thioalkoxy können die entsprechenden vorangehend aufgeführten Alkoxygruppen mit Schwefel statt Sauerstoff sein.

Das erfindungsgemäße Verfahren ist ein einfaches Verfahren zur enantioselektiven Synthese von (S)-1-Arylalkan-1-olen mit unerwartet hohen Enantiomerenüberschüssen. Hohe Enantiomerenüberschüsse sind im Sinne der Erfindung ee-Werte von größer oder gleich 80 % (≥ 80 %) (S-Enantiomeres), bevorzugt ee-Werte von größer oder gleich 90 % (≥ 90 %) (S-Enantiomeres), besonders bevorzugt von größer oder gleich 95 % (≥ 95 %) (S-Enantiomeres). Damit werden für die enantioselektive Synthese von (S)-1-Arylalkan-1-olen die bisher höchsten Enantiomerenüberschüsse erzielt. Alle bisher bekannten Versuche zur Darstellung dieser S-Enantiomeren verliefen mit deutlich niedrigeren ee-Werten.

Die oxygenasehaltigen Mikroorganismen werden vor der Umsetzung der oben genannten Substanzen auf komplexen oder mineralischen Nährmedien unter Anwendung von für die Anzucht der jeweiligen Mikroorgansimen üblichen Kulturverfahren (Kultivierung im Schüttelkolben, Batch-Fermentationen, Fed-Batch-Fermentationen bzw. kontinuierliche Fermentationen) bis zu einer optischen Dichte von 1 bis 500, bevorzugt von 5 bis 300, gemessen bei einer Wellenlänge von 600 nm (OD₆₀₀), angezogen und gegebenenfalls nach der Anzucht konzentriert.

Handelt es sich bei den oxygenasehaltigen Mikroorganismen um transformierte Mikroorganismen, so wird zunächst die natürliche, synthetische oder semi-synthetische Nukleinsäure gewonnen bzw. isoliert, gegebenenfalls aufgereinigt, mittels eines geeigneten, dem Fachmann bekannten Verfahrens kloniert und gegebenenfalls erneut aufgereinigt. Die isolierten Gene können dann in Zellen gleicher oder unterschiedlicher Art bezogen auf den Wildtyp mit geeigneten, dem Fachmann bekannten Methoden eingeführt werden. Die transformierten Wirtszellen werden dann wie im vorangehenden Abschnitt beschrieben angezogen.

Nachdem eine geeignete Zelldichte erreicht ist, erfolgt die Induktion der Genexpression durch Zugabe eines Induktormoleküls. Alternativ kann die Induktion auch durch Anwendung induzierender Kulturbedingungen bzw. Verwendung induzierend wirkender Medien erfolgen.

Für die Umsetzung wird das Edukt zu den Zellen gegeben, die entweder im Anzuchtmedium oder gegebenenfalls nach vorheriger Sedimentation in einer isotonischen Lösung resuspendiert vorliegen. Die Mischung aus Edukt und Zellen wird entweder durch Schütteln in einem Erlenmeyerkolben oder durch Belüftung in einer für die Belüftung von Zellsuspensionen geeigneten Anlage (z.B. in einem Fermenter) mit Sauerstoff versorgt.

Die Umsetzung kann entweder durch einmalige, mehrmalige oder kontinuierliche Eduktzugabe erfolgen. Die Eduktkonzentration in der Zellsuspension kann zwischen 1 und 900 mM, bevorzugt zwischen 2 und 500 mM liegen, besonders bevorzugt zwischen 3 und 250 mM liegen.

Zur Steigerung der Löslichkeit des Edukts während der Umsetzung können Hilfsstoffe wie z.B. Cyclodextrine oder DMSO (Dimethylsulfoxid) zugesetzt werden.

Die Umsetzung kann in einem pH-Bereich von 3 bis 11, bevorzugt von 4 bis 10, besonders bevorzugt von 5 bis 9 erfolgen. Sie wird üblicherweise bei einer Temperatur von 10 bis 60°C, vorzugsweise von 18 bis 45°C durchgeführt.

Nach der Umsetzung werden die entsprechenden (S)-1-Homo- oder (S)-1-Heteroarylalkan-1-ole unter Verwendung geeigneter Extraktionsmittel und -methoden aus der Zellsuspension isoliert. Bevorzugt werden hierzu handelsübliche Lösungsmittel wie Toluol, Ethylacetat, Dichlormethan, Isobutylketon, cyclo-Hexan, Methyl-cyclo-Hexan, u.a. eingesetzt. Die Extraktion kann dabei durch kontinuierliche oder diskontinuierliche Zuführ des Extraktionsmittels erfolgen. Im einfachsten Fall gelingt die Aufreinigung durch Ausschütteln mit den vorangehend erwähnten Extraktionsmitteln. Bevorzugt wird für das Ausschütteln Ethylacetat verwendet.

Unter vorangehend beschriebener Umsetzung ist im Sinne der Erfindung die enantioselektive Hydroxylierung von 1-Homo- oder 1-Heteroarylalkanen zu (S)-1-Homo- oder (S)-1-Heteroarylalkan-1-olen nach dem erfindungsgemäßen Verfahren zu verstehen.

Zum besseren Verständnis soll die Bedeutung bestimmter Wörter und Begriffe, die in der Beschreibung, den Beispielen und den Ansprüchen verwendet werden, im Folgenden näher erläutert werden.

Der Begriff "Vektor" beschreibt ein Nukleinsäure-Element, welches zum Einbringen exogener Nukleinsäuren in Wirtszellen verwendet wird. Ein Vektor enthält eine Nukleinsäuresequenz, die für ein oder mehrere Polypeptide kodiert. Vektoren, die in der Lage sind, die Expression der Gene zu steuern, welche sie enthalten, werden auch als "Expressionsvektoren" bezeichnet.

Der Begriff "Nukleinsäure" bezieht sich auf Polynukleotide wie Desoxyribonukleinsäure (DNA) oder, falls angebracht, auf Ribonukleinsäure (RNA). Der Begriff umfasst auch in äquivalenter Weise Analoga von RNA oder DNA, die aus Nukleotidanaloga hergestellt werden, sowie im zutreffenden Fall einzelsträngige ("Sense" und "Antisense") und doppelsträngige Polynukleotide.

Die Begriffe "Zelle" oder "Wirtszelle" können im Rahmen der vorliegenden Erfindung im gleichen Sinne verwendet werden. Es versteht sich, dass diese Begriffe nicht nur auf eine einzelne Zelle, sondern auch auf die Nachkommen solcher Zellen beziehen. Aufgrund bestimmter Modifikationen im Verlauf folgender Generationen (z.B. Mutationen) sind solche Nachkommen möglicherweise nicht mit der Stammzelle identisch, sind allerdings von der vorliegenden Erfindung mit umfasst.

Der Begriff "Promotor" bezieht sich auf Nukleinsäure-Sequenzen, welche die Expression einer bestimmten Nukleinsäure regulieren, die funktionell mit dem Promotor verknüpft ist, indem die Promotorsequenzen beispielsweise als spezifische Bindesequenz und Startstelle für die Transkription erkannt werden. Der Begriff umfasst auch "gewebsspezifische" Promotoren, d.h. solche, die die Expression der spezifischen Nukleinsäure nur in bestimmten Zellen (beispielsweise Zellen eines bestimmten Gewebes) steuern. Ebenso umfasst sind "gewebsunspezifische" Promotoren sowie Promotoren, die zu einer konstitutiven Expression führen oder induzierbar sind.

Der Begriff "Enhancer" bezieht sich auf Nukleinsäure-Sequenzen, welche die Expression einer bestimmten Nukleinsäure regulieren, indem sie die Aktivität der Promotoren steigern. Enhancer können sowohl die Expression homologer als auch heterologer Gene (eingeschleuster Fremdgene) stimulieren.

Die Begriffe "Klonierung" und "Klonieren" können im Rahmen der vorliegenden Erfindung im gleichen Sinne verwendet werden. Sie beschreiben das Isolieren und anschließende Vermehren von Nukleinsäure-Fragmenten bzw. Genen. Dies kann unter Anwendung der in der Molekularbiologie bzw. Gentechnik üblichen Methoden erfolgen; beispielsweise kann mittels PCR (polymerase chain reaction) zellfreie Klonierung erfolgen oder mit Hilfe eines Klonierungssystems kann über vielfältige Methoden ausgehend von Einschleusen der Fremd-DNA in Vektoren über Einbringen in eine Wirtszelle und anschließende Vermehrung der Wirtszellen eine zellgebundene Klonierung erfolgen.

Der Begriff "Amplifikation" bezieht sich auf die Erhöhung einer Gendosis durch Produktion zusätzlicher Kopien von Nukleinsäure-Fragmenten bzw. Genen, welche beispielsweise durch vorangehend beschriebene Klonierung oder durch die Durchführung einer PCR erzielt werden kann.

### Beispiele

"mM" steht vorangehend und im Folgenden für "millimolare" Lösungen (Konzentrationsangabe).

### Herstellung der Oxygenase-exprimierenden Mikororganismen

### 1. Präparation der genomischen DNA aus Pseudomonas putida G7

100 ml Luria-Bertani(LB)-Medium (10g/l Hefeextrakt; 5 g/l Peptone; 10 g/l NaCl; pH 7,1) wurden in einen 1000-ml-Erlenmeyerkolben gegeben, mit dem Bakterienstamm *Pseudomonas putida (P. putida)* G7 (DSM 4476) beimpft und über Nacht in einem Schüttelinkubator bei 30°C und 175 rpm inkubiert.

Die über Nacht gewachsene Kultur wurde in einer Zentrifuge bei 12.000 x g für 10 min sedimentiert. Der Überstand wurde verworfen und das Zell-Pellet in 40 ml Extraktionspuffer (100 mM Tris HCl (Trishydroxymethylaminomethan-Chlorid); 100 mM EDTA(Ethylendiamintetraacetat); 100 mM Na₂HPO₄/NaH₂PO₄ pH 8,0; 1,5 M NaCl; 1 % CTAB (Hexadecyl-trimethylammoniumbromid)) resuspendiert. Zum Proteinabbau wurden 140 µl Proteinase K (10 mg/ml) zugegeben. Die Proben wurden 30min bei 37°C im Wasserbad inkubiert und regelmäßigen Abständen geschüttelt. Es folgt die Zugabe von 2 ml SDS (Natriumdodecylsulfat) und eine erneute Inkubation für 2h bei 65°C im Wasserbad. Die Proben wurden wiederum in regelmäßigen Abständen geschüttelt.

Im Anschluss wurden die Proben drei mal bei -82°C im Ethanol/Trockeneisbad schockgefroren, im 65°C Wasserbad aufgetaut und für 16 min bei 8000 x g zentrifugiert. Der Überstand wurde dekantiert. Das Pellet wurde mit 10 ml Extraktionspuffer resuspendiert, für 30 min bei 60°C im Wasserbad inkubiert und zwischendurch geschüttelt. Nach erneuter Sedimentation der Zelltrümmer für 15min bei 8000 x g wurde der Überstand mit dem Überstand der ersten Extraktion vereinigt und die sedimentierten Zelltrümmer verworfen.

Die vereinigten Überstände wurden mit einem Volumen Chloroform/ Isoamylalkohol (24/1) ausgeschüttelt, die obere wässrige Phase vorsichtig abgehoben und zur Präzipitation der DNA mit dem 0,6fachen Volumen Isopropanol versetzt.

Diese Proben wurden gut geschüttelt und für 20min bei 18.000 x g zentrifugiert. Der Überstand wurde dekantiert und verworfen, während das DNA-Pellet zwei mal mit 20 ml eiskaltem 70 %igem Ethanol gewaschen wurde. Nach Zugabe des Ethanols wurden die Röhrchen kräftig geschüttelt. Anschließend wurden die Proben erneut für 10min bei 8000 x g zentrifugiert. Der Überstand wurde dekantiert und verworfen. Die DNA-Pellets wurden luftgetrocknet, bis kein Ethanolgeruch mehr wahrzunehmen war und in 8ml TE Puffer (10mM TrisHCl, 1mM EDTA, pH 8,0) aufgenommen. Zum Abbau der RNA wird die gelöste DNA mit 40µl Ribonuklease A-Lösung (100mg/ml) versetzt und für 15min bei 37°C im Wasserbad inkubiert. Nach der RNAse Behandlung wird die DNA-Lösung auf Eis gekühlt und bei -20°C gelagert.

### 2. Aufreinigung der genomischen DNA durch Agarosegel-Elektrophorese

400 µl der DNA Präparation wurden mit 10 µl Blaumarker (BlueJuice™, GibcoBRL, Karlsruhe) versetzt und auf ein präparatives 1,0 %iges Agarosegel aufgetragen und für 1 h bei 100 V aufgetrennt. Nach Beendigung der Auftrennung wurde die DNA-Bande in Form kleiner Würfel mit einem sterilen Skalpell aus dem Gel geschnitten. Ungefähr 100 mg DNA-hältiger Agarose wurden in einen ,Gel Nebulizer' (Millipore, Eschborn) gegeben.

Die mit Agarosegel-Stückchen beschickten ,Gel Nebulizer' wurden in ,Ultrafree-MC® Centrifugal Filter' (Millipore, Eschborn) überführt und in einem Eppendorf-Gefäß 10min bei 15.000 x g zentrifugiert. Die im Eppendorf-Gefäß ausgefangene eluierte DNA-Lösung wurde bei -20°C gelagert.

### 3. Klonierung der PCR-amplifizierten Oxygenasegene

Die für die Naphthalin-Dioxygenase kodierenden Gene (nahAa, nahAb, nahAc, nahAd, im Folgenden als nahAa-d zusammengefaßt) wurden mithilfe der Polymerasekettenreaktion (engl.: polymerase chain reaction = PCR) amplifiziert. Die genomische DNA des Wildtypstamms (*Pseudomonas putida* G7) diente als "template DNA". Als "primer" wurden folgende synthetisch hergestellten einzelsträngigen DNA-Moleküle eingesetzt:

‚forward primer‘: 5‘- GAATTCATGGAACTTCTCATCATACAGCCAAAC -3‘
(SEQ ID NO: 1)

‚reverse primer‘: 5‘- CTGCAGTCACAGAAAGACCATCAGATTGTG -3‘ (SEQ ID
NO: 2)

Die Sequenz des ,forward primer' entspricht der Anfangssequenz des nahAa-Gens und die des ,reverse primer' entspricht der Sequenz am Ende des nahAd-Gens. Um spätere Folge-Klonierungen leichter durchführen zu können, wurden dem ,forward primer' eine EcoRI-Restriktionsschnittstelle und dem ,reverse primer' eine Pstl-Restriktionschnittstelle angefügt (Restriktionsstellen jeweils unterstrichen).

Für die PCR-Amplifikation wurde auf Eis folgendes Reaktionsgemsich in ein 1,5-ml-Eppendorfgefäß pipettiert: 39 µl H₂O, 1 µl 50 mM MgSO₄, 1 µl genomische DNA-Lösung, 1 µl dNTP-Lösung (je 10 mM), 1 µl ,forward primer'-Lösung (100 ng/µl), 1 µl ,reverse primer'-Lösung (100 ng/µl).

Die PCR Ansätze wurden gründlich gemischt und in einem Thermocycler (Biometra T3 Thermocycler, Biometra, Göttingen) nach folgenden Temperatur-Programm inkubiert: 1 Zyklus: 120 s bei 94°C; 30 Zyklen: 30 s bei 94°C, 30 s bei 60°C, 210 s bei 68°C; 1 Zyklus 210 s bei 68°C; Temperatur halten bei 4°C.

### 4. Aufreinigung des PCR-Produkts

Die mittels PCR hergestellten DNA-Fragmente wurden in einem Agarosegel aufgetrennt und mithilfe des ,MinElute™ Gel Extraction Kit' (Qiagen, Hilden) aus dem Gel extrahiert.

Je 50 µl der PCR-Reaktion wurden mit je 5 µl ,10x BluejuiceTM' (GibcoBRL, Karlsruhe) versetzt und auf ein 1 %iges Agarosegel aufgetragen und für 1 h bei 100 V aufgetrennt. Nach Beendigung der Elektrophorese wurden die DNA-Fragmente mit einem sterilen Skalpell aus dem Gel geschnitten, die Gelbanden in ein Eppendorfgefäß überführt und mit 300 µl Puffer QG (Mat.No.1007226, Qiagen, Hilden) pro 100 mg Gel versetzt. Nach 10 min Inkubation bei 50°C inkubiert und regelmäßigem Mischen wurden den Ansätzen je 1 Vol Isopropanol zugeben und vorsichtig vermischt.

Im Anschluss wurde die DNA-Lösung in ,MinElute™ Spin Columns' (Qiagen, Hilden) überführt und 1 min bei 13.000 xg zentrifugieren.

Das Eluat wurde verworfen. In einem zweiten Schritt wurden 500 µl Puffer QG auf das ,MinElute™ Spin Columns' gegeben und für 1 min bei 13.000 xg zentrifugieren. Das Eluat wurde wiederum verworfen. Auf die gleiche Weise wurde das ,MinElute™ Spin Columns' noch einmal mit 750 µl Puffer PE (Mat.No.1015211, Qiagen, Hilden) beschickt und zweimal für 1 min bei 13.000 x g zentrifugiert.

Zum Auffangen der gereinigten DNA-Lösung wurde das ,MinElute™ Spin Columns' in ein frisches Eppendorfgefäß überführt und die gebundene DNA mit 10 µl Puffer EB (10mM TrisHCl, pH 8,5) eluiert.

### 5. Herstellung des Expressionstamms E. coli BL21(DE3)::pCR T7LCT-TOPO:: nahAa-d

Die PCR-Fragmente wurden unter Verwendung des ,pCR®T7/CT-TOPO®-TA Expression Kit ' (Invitrogen, Karlsruhe) in den ,pCR®T7/CT-TOPO®-Vektor' kloniert und in *Escherichia coli* (*E. coli*)-Zellen transformiert. Für die Ligation des PCR-Produkt mit dem ,pCR®T7/CT-TOPO®-Vektor' wurde folgende Reaktion angesetzt, gemischt und für 10 min bei Raumtemperatur (vorangehend und im Folgenden 23°C) inkubiert: 4 µl DNA-Lösung mit gereinigtem PCR-Fragment (siehe 4.), 1 µl Vektor-DNA-Lösung (,pCR®T7/CT-TOPO®-TA Expression Kit', Invitrogen, Karlsruhe), 1 µl Salz-Lösung (,pCR®T7/CT-TOPO®-TA Expression Kit', Invitrogen, Karlsruhe).

Für die Transformation der ligierten DNA in den Bakterienstamm *E. coli* DH5α wurden 5 µl des vorangehend beschriebenen Ligationsansatzes mit 50 µl Max Efficiency *E. coli* DH5α kompetenten Zellen (Fa. Invitrogen, Karlsruhe) in 15 ml sterilen Falcon-Röhrchen vermischt, 30 min auf Eis inkubiert, für 30 s auf 42°C erhitzt und nochmals für 2 min aus Eis gestellt.

Zur Regeneration wurden 450 µl SOC Medium (Glucose 18 g, Tryptone 20 g, Hefeextract 5 g, NaCl 0,5 g, deionisiertes H₂O 950 ml, GibcoBRL, Karlsruhe) zu den Zellen gegeben und diese für 1 h bei 37°C inkubiert. Zur Selektion wurden 250 µl der Zellsuspension auf festes LuriaBertani-Medium in Gegenwart von 100 µg/ml Ampicillin plattiert und über Nacht bei 30°C kultiviert.

Aus mehreren Klonen wurde die Plasmid-DNA unter Verwendung des Plasmid-Mini-Präparations-Kits (Qiagen, Hilden) isoliert, die Sequenz überprüft und die rekombinante Vektor-DNA nach dem oben beschrieben Verfahren in kompetente *E. coli* BL21(DE3)-Zellen (Stratagen, Heidelberg) transformiert.

Der resultierende Expressions-Stamm wurde als *E. coli* BL21(DE3)::pCRT7/CT-TOPO: :nahAa-d oder kurz als *E. coli* T7-NDO bezeichnet.

### 6. Herstellung des Expressionstamms E. coli BL21(DE3)::pVLT33::nahAa-d

Die Vektoren aus pCRT7/CT-TOPO::nahAa-d und pVLT33 wurden mit den Restriktionsenzymen *EcoRI* (New England Biolabs, Frankfurt a.M.) und *HIND*III (New England Biolabs, Frankfurt a.M.) verdaut (jeweils 30 µl DNA; 3 µl Enzym HindIII; 3 µl Enzym EcoRI; 7 µl Puffer 3; 27 µl H₂O; Inkubation für 2 bis 3 h bei 37°C). Dabei wurde der pVLT33 linearisiert und das für die Naphthalin-Dioxygenase kodierende nahAa-d-DNA-Fragment aus pCRT7/CT-TOPO::nahAa-d herausgeschnitten.

Die im Restriktionsverdau erhaltenen DNA-Fragmente wurden elektrophoretisch im Agarosegel aufgetrennt. Die DNA-Fragmente, die für den pVLT33-Vektor und das Naphthalin-Dioxygenasegen kodieren wurden unter Verwendung des QIAX II Agarose Gel Extraktion Kit (Qiagen, Hilden) in einem Agarosegel aufgetrennt, aus dem Gel isoliert (siehe 4.) und miteinander ligiert (15 µl pVLT33-DNA-Lösung, 6 µl nahAa-d-DNA-Lösung, 3 µl Ligasepuffer und 1 µl T4-Ligase (New England Biolabs, Frankfurt a.M.), Inkubation bei 16°C über Nacht).

Wie unter 5. beschrieben folgte eine Transformation in E. coli DH5α, eine Präparation der Plasmid-DNA verschiedener Klone, eine Sequenzüberprüfung und eine Transformation in kompetente *E. coli* BL21(DE3)-Zellen. Der resultierende Expressions-Stamm wurde als E. *coli* BL21(DE3)::pVLT33::nahAa-d bezeichnet.

### 7. Herstellung des Expressionstamms P. putida KT2440::pVLT33::nahAa-d

Zur Herstellung des Expressionsstamms *Pseudomonas putida* KT2440:: pVLT33:: nahAa-d wurde der pVLT33::nahAa-d-Vektor in kompetente *P. putida* KT2440-Zellen elektrotransformiert.

Zur Präparation kompetenter *P. putida* KT2440-Zellen wurde eine 50 ml Vorkultur des Stamms in LB-Medium bei 25 °C über Nacht angezogen. Mit 5 ml der Vorkultur wurde eine 500-ml-Hauptkultur gleichen Mediums beimpft und bis zu einer optischen Dichte (600 nm) von 0,650 bei 25°C kultiviert. Die Zellen wurden 30 min auf Eis gekühlt, und anschließend für 30 min bei 0°C und 8000 x g sedimentiert. Der Überstand wurde verworfen und das Zellpellet in 250 ml sterilem eiskalten H₂O resuspendiert. Um die Zellen zu Waschen wurden die Zellen ein zweites Mal sedimentiert und erneut in 250 ml eiskaltem H₂O resuspendiert. Es folgten 4 weitere Sedimentations- und Resuspensionschritte nach denen die Zellen in 125 ml eiskaltem H₂O, 125 ml eiskaltem H₂O + 10 % Glycerin und zweimal in 500 µl eiskaltem H₂O + 10 % Glycerin resuspendiert wurden. Nach der letzten Resuspension wurden die Zellen zur Lagerung in 50-µl-Portionen bei -80°C eingefroren.

Für die Elektrotransformation wurden 2 µl der pVLT33::nahAa-d-DNA in einer 0,1-cm-Elektroporationküvette (Bio-Rad, München) auf Eis mit einer 50-µl-Portion kompetenter *P. putida* KT2440-Zellen vermischt, die zuvor auf Eis aufgetaut worden waren. Die Elektrotransformation erfolgte bei 25kV, in dem Elektroporator 2510 (Eppendorf, Hamburg).

Im Anschluss wurden die Zellen sofort in 950 µl SOC-Medium (GibcoBRL, Karlsruhe) aufgenommen, 1 h bei 30°C regeneriert, danach zur Selektion auf festes Luria-Bertani-Medium mit 100 µg/ml Kanamycin plattiert und bei 30°C inkubiert.

### Beispiel 1: Oxidation verschiedener Arylalkane durch dem Stamm E. coli BL21(DE3)::pCRT7/CT-TOPO::nahAa-d

Der Stamm *E. coli* BL21/DE3)::pCRT7/CT-TOPO::nahAa-d wurde über Nacht bei 30°C in 20ml Luria-Bertani-Medium mit 100 µg/ml Ampicillin angezogen. Mit dieser Vorkultur wurden 200-ml Luria-Bertani-Medium und 500 µg/ml Ampicillin in einem 1-1-Erlenmeyerkolben beimpft und unter Schütteln ebenfalls bei 30°C inkubiert. Nach Erreichen einer optischen Dichte von 2, die bei 600 nm gemessen wurde (OD₆₀₀), wurde die Expression der nahAa-d-Gene durch Zugabe von 100 µl einer 1M IPTG-Lösung (IPTG = Isopropylthiogalactosid) induziert. Nach ca. 3 h erreichte die Kultur eine OD₆₀₀ von 4 und wurde durch 10 min Zentrifugation bei 10.000 x g geerntet und die Zellen wurden in einem 1/20 Volumen M9-Medium (200ml 5x konz. M9-Salze (Na₂HPO₄ x 7 H₂O 64g, KH₂PO₄ 15g, NaCl 2,5g, NH₄Cl 5g); 2ml 1M MgSO₄; 0,1ml 1M CaCl₂; 0,1ml 0.15 % Glycerin; 750 ml H₂O) suspendiert.

Für die Umsetzungsreaktion wurden je 1 ml der konzentrierten Zellsuspension in ein 13-ml-Schraubendeckelröhrchen überführt und mit 20 µl einer 200 mM ethanolischen Stammlösung des jeweiligen Eduktes (vgl. Tab.1) versetzt. Die Inkubation der Ansätze erfolgte bei 30°C auf einem Schüttler. Durch Zugabe von 1 ml Ethylacetat wurden die Reaktionen unmittelbar nach Start der Reaktion (sog. 0 h-Wert) und nach 17h gestoppt. Die Ansätze wurden intensiv ausgeschüttelt und nach einer Phasentrennungs-Zentrifugation (4000 x g) wurde die obere Ethylacetatphase abgehoben und in ein Probenröhrchen überführt. Zur besseren Phasentrennung wurden 400 µl 5M NaCl zugesetzt.

Die Ethylacetat-Phasen wurden mit Hilfe gekoppelter Gaschromatographie/Massenspektroskopie (GC-MS) und chiraler Gaschromatographie analysiert. Die Ergebnisse sind in Tabellel aufgeführt.

**Tab. 1:**

| **Hydroxylierung von Arylalkanen durch rekombinante Bakterien** | | | | | |
|---|---|---|---|---|---|
| **Edukt** | **Konz. [mM]** | **Reaktionszeit [h]** | **Produkt** | **Umsatz [%]** | **ee-Wert [%] (Enantiomer)** |
| Ethylbenzol | 4 | 17 | 1-Phenylethan-1-ol | 48,9 | > 98 (S) |
| 1-Ethyl-4-trifluormethylbenzol | 4 | 17 | 1-(4-Trifluorphenyl)ethan-1-ol | 20,2 | > 98 (S) |
| 1-Ethyl-4-fluorbenzol | 4 | 17 | 1-(4-Fluorphenyl)ethan-1-ol | 9,6 | > 98 (S) |
| 4-Chlor-1-ethylbenzol | 4 | 17 | 1-(4-Chlorphenyl)ethan-1-ol | 76,5 | > 98 (S) |
| Propylbenzol | 4 | 17 | 1-Phenylpropan-1-ol | 3,9 | > 98 (S) |
| 2-Brom-1-ethylbenzol | 4 | 17 | 1-(2-Brom-Phenyl)ethan-1-ol | 24,2 | > 98 (S) |

Die Edukte sind käuflich erhältlich.

### Beispiel 2: Oxidation von 4-Chlor-1-ethylbenzol durch die Stämme E. coli::pVLT33::nahAa-d und P. putida::pVLT33::nahAa-d

Anzucht, Expression der Naphthalin-Dioxygenase, die Umsetzung des 4-Chlor-1-ethylbenzols und die Analytik wurden mit den Expressionsstämmen *E. coli* BL21(DE3):: pVLT33:: nahAa-d und *P. putida* KT2440::pVLT33::nahAa-d wie im Beispiel 3 beschrieben durchgeführt. 4-Chlroethylbenzol wurde den Ansätzen in Konzentrationen von 4 bzw. 8 mM aus einer 400mM Stammlösung in Ethanol zugesetzt. Abweichend von Beispiel 1 erfolgte die Probenaufbereitung für die Analytik durch Zugabe von 1 Vol. Acetonitril.

**Tab. 2:**

| **Hydroxylierung von 4-Chlor-1-ethylbenzol durch rekombinante Bakterien** | | | | | |
|---|---|---|---|---|---|
| **Stamm** | **Konz. [mM]** | **Reaktionszeit [h]** | **Edukt/Produkt** | **Umsatz [%]** | **ee-Wert Produkt [%]** |
| *E. coli* BL21(DE3)::pVLT33::NDO | 4 | 17 | 4-Chlor-1-ethylbenzol/ 1-(4-Chlorphenyl)ethan-1-ol | 59,0 | > 98 (S) |
| *E. coli* BL21(DE3)::pVLT33::NDO | 8 | 17 | 4-Chlor-1-ethylbenzol/ 1-(4-Chlorphenyl)ethan-1-ol | 11,7 | > 98 (S) |
| *P*. *putida* KT2440::pVLT33::NDO | 4 | 17 | 4-Chlor-1-ethylbenzol/ 1-(4-Chlorphenyl)ethan-1-ol | 21,8 | > 98 (S) |
| *P. putida* KT2440::pVLT33::NDO | 8 | 17 | 4-Chlor-1-ethylbenzol/ 1-(4-Chlorphenyl)ethan-1-ol | 23,9 | > 98 (S) |

## Patentansprüche

1. Verfahren zur Herstellung von (S)-1-Homo- oder (S)-1-Heteroarylalkan-1-olen durch enantioselektive Hydroxylierung von 1-Homo- oder 1-Heteroarylalkanen, **dadurch gekennzeichnet, dass** die Hydroxylierung in Gegenwart eines Mikroorganismus durchführt wird, enthaltend Nukleinsäuren, die für eine Oxygenase kodieren, welche die enantioselektive (S)-Hydroxylierung katalysiert.

2. Verfahren nach Anspruch 1, **dadurch gekennzeichnet, dass** die Hydroxylierung in Gegenwart eines transformierten Mikroorganismus durchführt wird, enthaltend Nukleinsäuren, die für eine Oxygenase kodieren.

3. Verfahren nach Anspruch 1 oder 2, **dadurch gekennzeichnet, dass** die Hydroxylierung in Gegenwart von Bakterien, Hefen oder Pilzen durchgeführt wird, enthaltend Nukleinsäuren, die für eine Oxygenase kodieren.

4. Verfahren nach wenigstens einem der Ansprüche 1 bis 3, **dadurch gekennzeichnet, dass** die Hydroxylierung in Gegenwart von Gram-negativen Bakterien durchgeführt wird.

5. Verfahren nach wenigstens einem der Ansprüche 1 bis 4, **dadurch gekennzeichnet, dass** die Hydroxylierung in Gegenwart von Gram-negativen Bakterien der Gattung *Pseudomonas, Rhodopseudomonas, Burkholderia, Ralstonia, Comamonas, Acinetobacter, Rhizobium* oder *Escherichia coli* durchgeführt wird.

6. Verfahren nach wenigstens einem der Ansprüche 1 bis 5, **dadurch gekennzeichnet, dass** die Hydroxylierung in Gegenwart eines *Escherichia coli*-K 12-Stammes oder eines *Pseudomonas putida*-Stammes durchgeführt wird.

7. Verfahren nach wenigstens einem der Ansprüche 1 bis 6, **dadurch gekennzeichnet, dass** die Mikroorganismen Nukleinsäuren enthalten, die für eine Naphthalin-Dioxygenase kodieren.

8. Verfahren nach wenigstens einem der Ansprüche 1 bis 7, **dadurch gekennzeichnet, dass** die Mikroorganismen Nukleinsäuren enthalten, die für eine Naphthalin-Dioxygenase aus *Pseudomonas putida G7* (DSM 4476) oder *Pseudomonas sp.* NCIB 9816 (DSM 8368) oder ähnliche Enzyme mit mindestens 70 % Identität über die Gesamtlänge der Aminosäuresequenz kodieren.

9. Verfahren nach wenigstens einem der Ansprüche 1 bis 8, **dadurch gekennzeichnet, dass** als Edukt (C₁-C₆)-Alkyl-(C₅-C₁₄)-aromaten verwendet werden, die einfach oder mehrfach, gleich oder verschieden ringsubstituiert sind und worin 0 bis 3 Kohlenstoffatome durch Heteroatome aus der Gruppe N, O und/oder S im aromatischen Ringsystem ersetzt sind.

10. Verfahren nach wenigstens einem der Ansprüche 1 bis 9, **dadurch gekennzeichnet, dass** als Edukt (C₁-C₆)-Alkyl-(C₅-C₆)-aromaten verwendet werden, die einfach oder mehrfach gleich oder verschieden ringsubstituiert sind und worin 0 bis 3 Kohlenstoffatome durch Heteroatome aus der Gruppe N, O und/oder S im aromatischen Ringsystem ersetzt sind.

11. Verfahren nach wenigstens einem der Ansprüche 1 bis 10, **dadurch gekennzeichnet, dass** (S)-1-Homoarylalkan-1-ole der allgemeinen Formel (I), 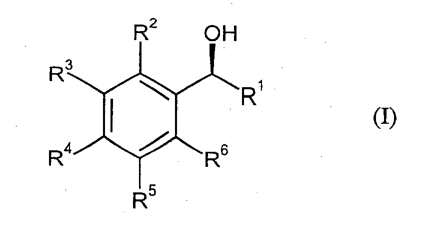 wobei
R¹ für C₁-C₃-Alkyl steht,
R², R³, R⁴, R⁵ und R⁶ unabhängig voneinander für H, Halogen, C₁-C₄-Halogenalkyl, C₁- C₄-Alkyl, Hydroxy, C₁-C₄-Alkoxy, Thiol, C₁-C₄-Thioalkoxy, Amino, primäres oder sekundäres C₁-C₄-Aminoalkyl stehen und mindestens ein Rest R² bis R⁶ von H verschieden ist,
durch enantioselektive Hydroxylierung von Verbindungen der allgemeinen Formel II, 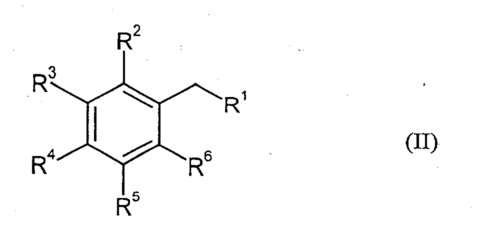 wobei
R¹ die für Formel I genannte Bedeutung hat und
R², R³, R⁴, R⁵ und R⁶ unabhängig voneinander die für Formel I genannte Bedeutung haben,
hergestellt werden.

12. Verfahren nach Anspruch 11, **dadurch gekennzeichnet, dass** R¹ für C₁-C₃-Alkyl und R², R³, R⁴, R⁵ und R⁶ unabhängig voneinander für H, Halogen, C₁-C₄-Halogenalkyl stehen und mindestens ein Rest R² bis R⁶ von H verschieden ist.

13. Verfahren nach wenigstens einem der Ansprüche 11 oder 12, **dadurch gekennzeichnet, dass** R¹ für Methyl steht.


SEQUENZPROTOKOLL

<110> Bayer Aktiengesellschaft

<120> Mikrobiologisches Verfahren zur enantioselektiven (S)-
Hydroxylierung

<130> Le A 35 826

<160> 2

<170> PatentIn version 3.1

<210> 1
<211> 33
<212> DNA
<213> Pseudomonas putida

<400> 1

gaattcatgg aacttctcat catacagcca aac
33

<210> 2
<211> 30
<212> DNA
<213> Pseudomonas putida

<400> 2

ctgcagtcac agaaagacca tcagattgtg
30